# EUROPÄISCHE PATENTANMELDUNG

(11) **EP 3 178 389 A1**
(43) Veröffentlichungstag der Anmeldung: **14.06.2017**
(21) Anmeldenummer: 16203265.0
(22) Anmeldetag: 09.12.2016
(51) Int. Cl.: A61B 5/04, A61B 5/00, A61B 5/0488, A61B 17/00

(54) **DETEKTIONSVORRICHTUNG**

(30) Priorität: 10.12.2015 DE 102015224879
(71) Anmelder: Deutsches Zentrum für Luft- und Raumfahrt e.V., 51147 Köln (DE)
(72) Erfinder: Urbanek, Holger, 80687 München (DE); Schwier, Andrea, 80634 München (DE)
(74) Vertreter: dompatent von Kreisler Selting Werner - Partnerschaft von Patent- und Rechtsanwälten mbB

(57) **Zusammenfassung**

Eine Detektionsvorrichtung zum Detektieren von Nervenfasern weist mindestens zwei Elektroden zum Messen eines Aktionspotentials einer zu detektierenden Gewebefaser und eine Frequenzanalysevorrichtung auf. Letztere ist ausgebildet zum Feststellen einer Häufigkeit und/oder einer Stärke von Aktionspotentialen in verschiedenen Frequenzbereichen. Basierend auf der Häufigkeit und7oder Stärke von Aktionspotentialen in einem bestimmten Frequenzbereich wird auf das Vorhandensein einer Nervenfaser geschlossen.

## Beschreibung

Die Erfindung betrifft eine Detektionsvorrichtung zur Detektion von Nervenfasern.

Eine derartige Vorrichtung kann beispielsweise während einer Operation an einem Patienten verwendet werden, um Nervenfasern von anderen Fasern, beispielsweise Muskelfasern, zu unterscheiden. Hierdurch kann das Risiko einer Nervenverletzung beziehungsweise Nervendurchtrennung verringert werden.

Aus dem Stand der Technik sind Schnittbildverfahren wie die Magnetresonanztomographie und die Sonographie bekannt, durch die eine direkte Darstellung des peripheren Nervensystems ermöglicht wird.

Ein weiteres bekanntes Verfahren ist die optische Kohärenztomographie (OCT), die eine Darstellung verschiedener anatomischer Strukturen ermöglicht, zum Beispiel des Auges (dort unter anderem des Sehnervs).

Bei der optischen Kohärenztomographie können nur Strukturen bis zu einer Tiefe von ca. 2 mm dargestellt werden.

Die Magnetresonanztomographie ermöglicht eine Darstellung der Nerven nur vor einem operativen Eingriff. Durch die Umlagerung des Patienten verändert sich jedoch die Position des Nervenstranges, so dass keine genaue Aussage über den Verlauf des Nervs getroffen werden kann.

Bei der Sonographie besteht das Problem, dass tiefer liegende Strukturen nur mit schlechterer Auflösung oder gar nicht dargestellt werden können. Auch sind die Nervenfasern nicht sofort erkennbar, sondern nur durch Erfahrung des Bedieners.

Die bekannten Verfahren ermöglichen keine zuverlässige Aussage über den Verlauf von Nervenfasern während eines operativen Eingriffs. Die Unversehrtheit der Nervenfasern ist allein vom Wissen und der Erfahrung des Operateurs abhängig.

Aufgabe der Erfindung ist es, eine Detektionsvorrichtung bereitzustellen mit Hilfe derer Nervenfasern insbesondere während eines chirurgischen Eingriffs einfach und zuverlässig detektiert werden können.

Die Lösung der Aufgabe erfolgt erfindungsgemäß durch die Merkmale des Anspruchs 1.

Die erfindungsgemäße Detektionsvorrichtung zur Detektion von Nervenfasern weist eine oder mehrere Elektroden zum Messen von Aktionspotentialen einer zu detektierenden Gewebefaser auf. Die Elektroden können derart ausgestaltet sein, dass sie auf der Haut des Patienten platziert werden und von dort aus die Aktionspotentiale von im Körper befindlichen Gewebefasern messen. Hierzu werden die Elektroden in einer Art und Weise eingesetzt, wie sie bereits aus elektromyographischen Verfahren bekannt ist.

Alternativ können die Elektroden derart ausgestaltet sein, dass sie direkt beziehungsweise in unmittelbarer Nähe zu der zu detektierenden Gewebefaser Aktionspotentiale messen. Hierzu können die Elektroden beispielsweise auch als Nadeln ausgebildet sein, die in den Patientenkörper eingeführt werden, bis sie sich in der Nähe oder unmittelbar an der zu detektierenden Gewebefaser befinden. In diesem Fall werden insbesondere bei Nervenfasern wesentlich stärkere Aktionspotentiale erfasst. Die Aktionspotentiale unmittelbar an einer Nervenfaser können beispielsweise im Bereich von 90 bis 120 mV liegen. Dagegen haben die Aktionspotentiale an der Nervenfaser bei einer Messung auf der Haut des Patienten im besten Falle eine Stärke von ca. 1 bis 100 µV. Auch Muskelfasern weisen bei einer Messung unmittelbar an der Faser Aktionspotentiale von beispielsweise 120 mV auf. Die Signalstärke bei Muskelfasern nimmt bei einer Messung auf der Haut weniger stark ab, so dass die Signale immer noch in einem Bereich zwischen 1 und 100 mV liegen können. Die genannten Werte beziehen sich auf eine Messart, die als "Maximal Voluntary Contraction" bezeichnet wird.

Die erfindungsgemäße Vorrichtung ist gekennzeichnet durch eine Frequenzanalysevorrichtung, die ausgebildet ist zum Feststellen einer Häufigkeit, Form und/oder Stärke von Aktionspotentialen in verschiedenen Frequenzbereichen. Basierend auf der Energie, entsprechend dem Integral über einen Frequenzbereich, innerhalb eines bestimmten Nervenfaser-Frequenzbereichs wird auf das Vorhandensein einer Nervenfaser geschlossen.

Durch das letztgenannte Merkmal unterscheidet sich die erfindungsgemäße Vorrichtung von bisher bekannten Vorrichtungen, die für die Elektromyographie eingesetzt wurden.

Vorzugsweise wird die Energie innerhalb eines Nervenfaser-Frequenzbereichs in einem ersten Bereich des Gewebes mit der Energie innerhalb desselben Nervenfaser-Frequenzbereichs in einem zweiten möglicherweise benachbarten Bereich des Gewebes verglichen. Sofern die Energie im ersten Bereich des Gewebes höher ist als die Energie im zweiten Bereich des Gewebes, kann davon ausgegangen werden, dass im ersten Bereich des Gewebes eine Nervenfaser vorliegt.

Durch die erfindungsgemäße Vorrichtung ist es möglich, auf einfache Weise das Vorhandensein von Nervenfasern auch während einer Operation zu detektieren. Hierfür muss der Patient nicht umgebettet werden, so dass der Operateur sichergehen kann, dass die detektierte Nervenfaser sich im weiteren Verlauf der Operation an derselben Stelle befindet, an der sie durch die erfindungsgemäße Vorrichtung detektiert wurde. Abhängig vom Ergebnis, das durch die erfindungsgemäße Vorrichtung geliefert wird, kann der Operateur seine weiteren Handlungen anpassen, so dass eine Verletzung von Nervenfasern während der Operation verhindert werden kann. Mit anderen Worten kann die erfindungsgemäße Vorrichtung somit dem Chirurgen während der Operation Hinweise dazu geben, ob ein geplanter Schnitt gefahrlos durchgeführt werden kann. Dies kann nicht nur während der Planung der Operation, sondern auch schon am geöffneten Patienten erfolgen, wenn beispielsweise Schnitte vertieft werden sollen. Die erfindungsgemäße Vorrichtung kann somit nicht lediglich auf der Hautoberfläche, sondern auch bei Gewebe innerhalb des Patientenkörpers angewendet werden.

Der Unterschied zur üblichen elektromyographischen Verwendung von Elektroden besteht somit darin, dass durch eine Frequenzanalyse des gewonnenen Signals Nervenfasern von Muskelfasern und anderen signalleitenden Fasertypen unterschieden werden können. Dies liegt darin begründet, dass sich die chemischen Eigenschaften von Nervenfasern von denjenigen anderer Fasertypen unterscheiden und somit die Frequenzzusammensetzung der Aktionspotentiale, die von Nervenfasern ausgehen, unterschiedlich ist.

Die unterschiedlichen Aktionspotentiale von Nervenfasern im Gegensatz zu anderen Fasertypen sind aus dem Stand der Technik bekannt und können beispielsweise der folgenden Veröffentlichung entnommen werden: Jens Huppelsberg, Kerstin Walter, "Kurzlehrbuch Physiologie", Georg Thieme Verlag, 3. Auflage, 2009, Seite 230.

Die erfindungsgemäße Vorrichtung ist bevorzugt dadurch gekennzeichnet, dass durch die Frequenzanalysevorrichtung ein Detektieren einer Nervenfaser erfolgt, wenn der Anteil der von den Aktionspotentialen stammenden Frequenzen zwischen 200 und 5000 Hz insbesondere zwischen 500 und 2000 Hz im Vergleich zum umgebenden Gewebe erhöht ist. Weiterhin kann definiert werden, dass ein Aktionspotential erst dann berücksichtigt wird, wenn dessen Stärke einen bestimmten Schwellwert überschreitet. Dieser kann bei einer Nervenfaser bei einer Messung auf der Haut beispielsweise bei 1 µV beziehungsweise bei einer Messung unmittelbar an der Nervenfaser bei 10 mV liegen.

Weiterhin ist es möglich, dass durch die Frequenzanalysevorrichtung ein Detektieren einer Muskelfaser erfolgt, wenn der überwiegende Teil der Energie der Aktionspotentiale zwischen 1 und 1000 Hz, insbesondere 10 und 500 Hz, 10 und 200 Hz, 50 und 200 Hz oder 50 und 100 Hz erfasst wird. Auch hier ist es möglich, lediglich solche Aktionspotentiale zu berücksichtigen, deren Stärke einen bestimmten Schwellwert überschreitet. Dieser kann bei einer Messung auf der Haut beispielsweise bei 1 mV liegen, während bei einer Messung unmittelbar an der Gewebefaser ein Schwellwert von 10 mV verwendet werden kann.

Ein Detektieren einer Muskelfaser erfolgt bevorzugt dann, wenn keine Nervenfaser gemäß den bisher beschriebenen Merkmalen in einem bestimmten Bereich des Gewebes detektiert wurde. In diesem Fall kann davon ausgegangen werden, dass in diesem Gewebe keine Nervenfaser sondern lediglich Muskelfasern vorliegen.

Die erfindungsgemäße Vorrichtung kann eine Anzeigevorrichtung zur optischen Anzeige der Art der detektierten Gewebefaser aufweisen. Hierbei kann es sich beispielsweise um ein Display handeln. Alternativ kann die erfindungsgemäße Detektionsvorrichtung eine drahtlose oder drahtgebundene Schnittstelle zu einem externen Display aufweisen. Hierbei kann es sich beispielsweise um ein Display handeln, das ohnehin im Operationssaal vorhanden ist, beispielsweise das Display, das zur Bedienung eines minimalinvasiven Chirurgie-Roboters verwendet wird. Eine Darstellung der Informationen, die durch die erfindungsgemäße Vorrichtung gewonnen werden, auf einem solchen Display hat den Vorteil, dass der Operateur diese Informationen direkt im räumlichen Zusammenhang mit dem zu manipulierenden Gewebe wahrnehmen kann.

Es ist besonders bevorzugt, dass auf der optischen Anzeigevorrichtung eine farbliche Kodierung der Art der erfassten Gewebefasern dargestellt wird, so dass die Art der Fasern vom Chirurgen leicht unterschieden werden können. Beispielsweise ist es möglich, dass Gewebefasern, deren überwiegender Teil der Aktionspotentiale zwischen 0 und 200 Hz liegt, in einer ersten Farbe, beispielsweise rot, angezeigt werden. Gewebefasern, deren überwiegender Teil der Aktionspotentiale zwischen 200 und 500 Hz liegt, können in einer zweiten Farbe, beispielsweise in grün, angezeigt werden. Gewebefasern, deren überwiegender Teil der Aktionspotentiale zwischen 500 und 10.000 Hz liegt können in einer dritten Farbe, beispielsweise in blau, angezeigt werden. Ist der Bediener farbenblind, können auch andere Farbkombinationen gewählt werden, die für den Bediener besonders gut unterschieden werden können. Hierdurch kann der Chirurg auf besonders intuitive Weise die Art der detektierten Gewebefasern erkennen. Je mehr sich bei dem dargestellten Beispiel die angezeigte Farbe in Richtung blau verändert, desto größer wird die Gefahr, dass eine Nervenfaser detektiert wurde, da diese auch nennenswerte Anteile im Frequenzbereich oberhalb von 500 Hz aufweisen wird.

In einer vereinfachten Ausführungsform ist es auch möglich, lediglich zwei Frequenzbänder durch die Anzeigevorrichtung anzuzeigen. Beispielsweise kann ein erstes Frequenzband zwischen 0 und 500 Hz oder alternativ 1.000 Hz in einer ersten Farbe auf der Anzeigevorrichtung angezeigt werden, während ein zweites Frequenzband zwischen 500 und 10.000 Hz oder alternativ zwischen 1.000 und 10.000 Hz in einer zweiten Farbe angezeigt wird.

Es ist bevorzugt, dass die Darstellung der beteiligten Frequenzen direkt in räumlichem Zusammenhang mit dem jeweiligen Messpunkt auf der Anzeigevorrichtung erfolgt. Für eine Darstellung in rot, grün und blau wird das Signal in drei Frequenzbänder aufgeteilt, deren Amplitude jeweils den Rot-, Grün- und Blauwerten zugeordnet wird. Es ist bevorzugt, dass die Aufteilung der Frequenzbänder beziehungsweise deren Zuordnung zu der jeweiligen Farbe durch den Arzt anpassbar ist, so dass beispielsweise die oben genannten Frequenzbereiche veränderbar sind. Dies kann in Abhängigkeit von den jeweiligen Operationsbedingungen notwendig sein. Die Gewinnung der RGB-Werte kann auf den absoluten Amplituden oder aber auf einem Verhältnis dieser zueinander basieren. Weiterhin kann entweder das Maximum oder der Durchschnitt oder aber auch ein anderes Maß innerhalb des Frequenzbandes zu einer Maßzahl innerhalb dieses Frequenzbandes führen, welche dann zu einem Rot-, Grün- oder Blauwert führt. Andere Farbräume beispielsweise HSV oder auch die Verwendung von mehreren Frequenzbändern sind ebenfalls möglich.

Die Anzeige kann auch beispielsweise auf einer Augmented-Reality-Brille oder alternativ auf einem Display direkt oberhalb der Elektroden erfolgen. Bei der letztgenannten Möglichkeit wäre somit die Anzeige direkt an der erfindungsgemäßen Vorrichtung angebracht.

Bei bipolaren oder auch tripolaren Elektrodenkonfigurationen verändert der Abstand der beiden Pole die Aufzeichnungstiefe der Signale: Je weiter die beiden Pole voneinander entfernt sind, desto besser können tiefer liegende Signale noch aufgezeichnet werden. Bei einer geeigneten Anordnung von mehreren Elektroden können diese durch eine geeignete Elektronik so verschaltet werden, dass deren Abstand veränderbar ist und somit eine Tiefenabschätzung ermöglicht wird.

Alternativ kann bei genau bekannten Elektrodenpositionen die Tiefe und Lage der detektierten Gewebefasern rekonstruiert werden. Hierzu kann ein Verfahren verwendet werden, wie es beispielsweise aus DE 10 2012 211 799 B3 bekannt ist. Darin wird durch eine Simulation des Gewebes ein Gleichungssystem aufgestellt welches den Einfluss eines Volumenausschnitts auf jede EMG-Elektrode ausdrückt. Daraus ergibt sich dann ein Gleichungssystem, welches mittels einer geeigneten Regularisierung in Bezug auf diese Volumenelemente gelöst wird. Im Falle von DE 10 2012 211 799 B3 werden zur Regularisierung der Durchschnitt vieler zufälliger Partitionierungen der Volumenelementen verwendet.

Werden die Messungen durch einen Roboter oder durch ein anderes System ausgeführt, das die genaue Position der Elektroden auch bei mehrfachem Neuauflegen genau bestimmen kann, ist es möglich, eine Karte des Gewebes zu erstellen.

In der einfachsten Ausführungsform ist es möglich, unter Verwendung einer einzigen Elektrode eine monopolare Messung durchzuführen. Zusätzlich hierzu kann eine Vergleichsmessung durch eine Referenzelektrode durchgeführt werden, die entweder in derselben Vorrichtung angebracht ist oder aber an einer anderen Stelle im Patientenkörper angebracht, beispielsweise am Ohrläppchen.

Beim Einsatz der erfindungsgemäßen Vorrichtung während der Anästhesie muss darauf geachtet werden, dass die betroffenen Nervenfasern weiterhin Signale leiten können und ihrer Synapsen oder Neurotransmitter nicht blockiert werden. Grundsätzlich kann die erfindungsgemäße Vorrichtung ohne eine externe Anregung der Nervenfasern verwendet werden, da Nervenzellen typischerweise eine hohe Ruhefeuerrate von 10 bis 100 Impulsen pro Sekunde haben. Innerhalb einer vertretbaren Zeit können somit immer einige Impulse durch die zu detektierenden Nervenfasern aufgezeichnet werden.

Um das System zu kalibrieren, ist es möglich, ein Ruherauschen, das im Patientenkörper herrscht und beispielsweise durch Geräte oder Stromleitungen im Operationssaal induziert wird, vom gemessenen Signal abzuziehen. Dies kann beispielsweise dadurch erfolgen, dass eine Vergleichsmessung in einem Gewebebereich durchgeführt wird, in dem bekannt ist, dass sich dort nur Muskelfasern befinden. Die dort erfassten Signale werden als Grundrauschen identifiziert. Sodann wird durch die Vorrichtung eine Messung in einem möglicherweise benachbarten zweiten Gewebebereich durchgeführt. Sofern hier mehr Frequenzen im beschriebenen Nervenfaser-Frequenzbereich gemessen werden als in dem Muskelfasergewebe, wird vom Vorhandensein einer Nervenfaser an dieser Stelle ausgegangen. Zur weiteren Verbesserung des Signals ist es insbesondere möglich, das im Bereich der reinen Muskelfaser gemessene Grundrauschen von dem im zweiten Gewebebereich gemessenen Signal abzuziehen, so dass hierdurch eine Kalibrierung vorgenommen werden kann.

Das Durchführen einer Vergleichsmessung in einem Bereich des Gewebes von dem bekannt ist, dass hier nur Muskelfasern vorliegen, kann grundsätzlich bei allen Ausführungsformen der Erfindung Anwendung finden. In der Praxis wird somit ein Operateur die Vorrichtung in einem Gewebebereich platzieren, von dem er weiß, dass dort nur Muskelfasern vorhanden sind. Ausgehend von diesem Bereich kann er die Vorrichtung in verschiedene Richtungen bewegen. Wenn hierbei die Energie der gemessenen Aktionspotentiale im Nervenfaser-Frequenzbereich im Vergleich zu der ersten Messung im Muskelgewebe erhöht ist, wird der Operateur vom Vorliegen einer Nervenfaser ausgehen.

Es ist bevorzugt, dass die Grenzen der Frequenzbänder, durch die ein Nervenfaser-Frequenzbereich und ein Muskelfaser-Frequenzbereich definiert werden, vom Bediener veränderbar sind.

Weiterhin ist bevorzugt, dass die Gewichtung, mit der die Energien der unterschiedlichen Aktionspotentiale zu einer Änderung der Farbintensität und/oder Helligkeit in der beschriebenen Anzeige der Vorrichtung führen, durch den Benutzer veränderbar ist. Hierdurch kann ein Operateur basierend auf seiner Erfahrung die Funktion der erfindungsgemäßen Vorrichtung verbessern.

Sollten die Nervenfasern zu wenige Impulse aussenden, kann das zu detektierende Gewebe auch mittels externer Impulse angeregt werden. Beispielsweise können hier direkt Neurotransmitter appliziert werden oder die Nervenfaser auch direkt elektrisch gereizt werden.

Es ist bevorzugt, dass durch die Frequenzanalysevorrichtung eine Aufteilung der durch die Elektroden erfassten Aktionspotentiale in ihre Frequenzanteile erfolgt. Die Aufteilung kann insbesondere durch eine Fourier-Analyse, besonders bevorzugt mittels einer Fast Fourier Transformation erfolgen. Auch können Wavelets Verwendung finden, wobei dann der strikte Begriff der Frequenzanalyse aufgeweicht wird.

Weiterhin ist es bevorzugt, dass die Detektionsvorrichtung eine Vielzahl von Elektroden aufweist, einem sogenannten Elektrodenarray. Hierdurch ist es möglich, bei einer einzigen Messung einen größeren Gewebebereich abzudecken.

Im Folgenden werden bevorzugte Ausführungsformen der Erfindung anhand von Figuren erläutert.

Es zeigen:
- Figur 1a und 1b: eine beispielhafte Darstellung von zwei Elektrodenköpfen,
- Figur 2: eine beispielhafte Darstellung eines Szenarios, in dem die erfindungsgemäße Detektionsvorrichtung verwendet werden kann.

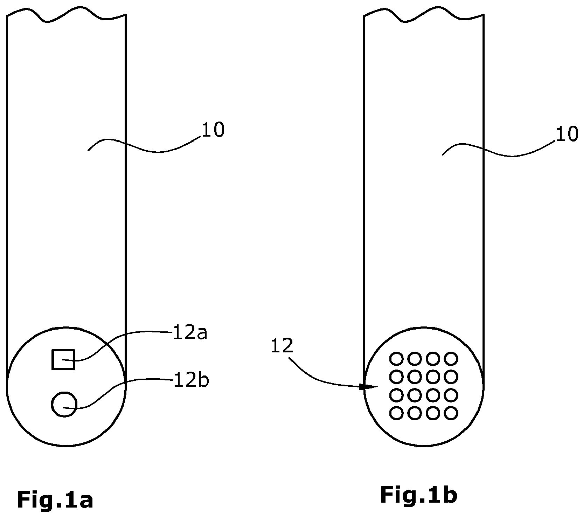

Figur 1a zeigt einen Elektrodenkopf 10 mit einer Messelektrode 12a und einer Referenzelektrode 12b.

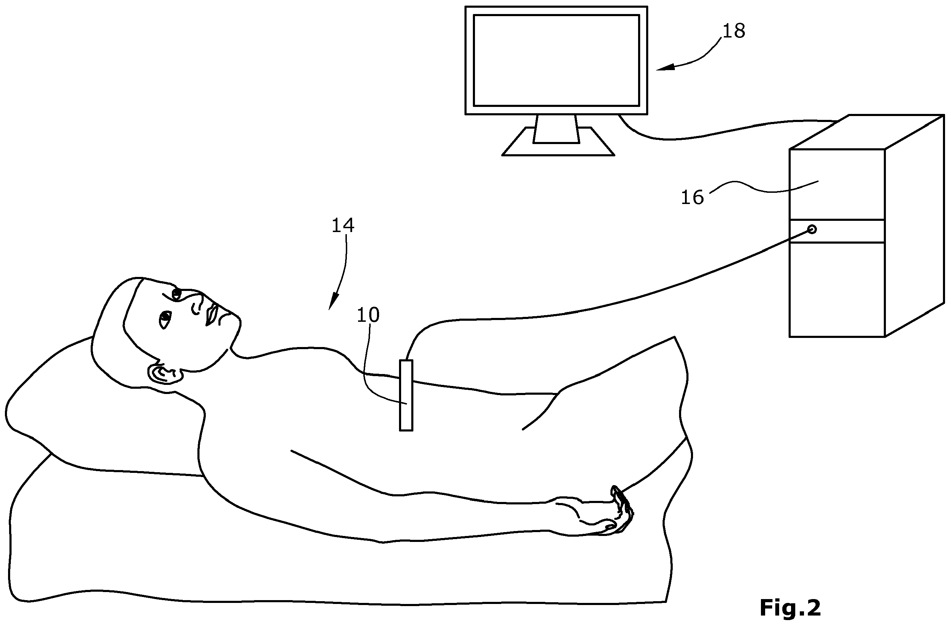

Der Elektrodenkopf 10 kann in einer Anordnung gemäß Figur 2 an der Haut des Patienten 14 angelegt werden, so dass das Aktionspotential einer zu detektierenden Gewebefaser gemessen werden kann. Hierzu kann als Frequenzanalysevorrichtung 16 beispielsweise ein PC verwendet werden, mit dem die Messelektrode 10 datentechnisch verbunden ist. Der PC 16 kann mit einem Monitor 18 verbunden sein.

Eine alternative Ausgestaltung eines Elektrodenkopfs 10 mit einem Elektroden Array 12, das eine Vielzahl von übereinander und nebeneinander angeordneten Elektroden aufweist, ist in Figur 1b dargestellt. Dieses kann ebenfalls in einem Szenario gemäß Figur 2 verwendet werden.

## Patentansprüche

1. Detektionsvorrichtung (10) zum Detektieren von Nervenfasern, wobei die Detektionsvorrichtung aufweist:
mindestens eine Elektrode (12a, 12b) zum Messen eines Aktionspotentials einer zu detektierenden Gewebefaser,
eine Frequenzanalysevorrichtung (16), die ausgebildet ist zum Feststellen einer Häufigkeit, Form und/oder Stärke von Aktionspotentialen in verschiedenen Frequenzbereichen,
wobei basierend auf der Energie in einem bestimmten Frequenzbereich auf das Vorhandensein einer Nervenfaser geschlossen wird.

2. Detektionsvorrichtung (10) nach Anspruch 1, **dadurch gekennzeichnet, dass** durch die Frequenzanalysevorrichtung (16) ein Detektieren einer Nervenfaser erfolgt, wenn im Frequenzbereich zwischen 10 und 5000 Hz, insbesondere zwischen 50 und 2000 Hz eine erhöhte Energie im Vergleich zum umliegenden Gewebe erfasst wird.

3. Detektionsvorrichtung (10) nach Anspruch 1 oder 2, **dadurch gekennzeichnet, dass** durch die Frequenzanalysevorrichtung (16) ein Detektieren einer Muskelfaser erfolgt, wenn der überwiegende Teil der Energie zwischen 1 und 1000 Hz, insbesondere 10 und 500 Hz, 10 und 200 Hz, 50 und 200 Hz oder 50 und 100 Hz erfasst wird und kaum Anteile in höheren Frequenzspektren vorhanden sind.

4. Detektionsvorrichtung (10) nach einem der Ansprüche 1 bis 3, **gekennzeichnet durch** eine Anzeigevorrichtung zur optischen Anzeige der Art der detektierten Gewebefaser.

5. Detektionsvorrichtung (10) nach Anspruch 4, **dadurch gekennzeichnet, dass** die Energie, die zwischen 0 und 200 Hz liegt, in einer ersten Farbe auf der Anzeigevorrichtung angezeigt werden,
die Energie, die zwischen 200 und 500 Hz liegt, in einer zweiten Farbe auf der Anzeigevorrichtung angezeigt werden,
die Energie, die zwischen 500 und 10.000 Hz liegt, in einer dritten Farbe angezeigt werden.

6. Detektionsvorrichtung (10) nach Anspruch 5, **dadurch gekennzeichnet, dass** die Grenzen der Frequenzbänder vom Bediener verändert werden können.

7. Detektionsvorrichtung (10) nach Anspruch 5, **dadurch gekennzeichnet, dass** die Energien, die zur Farbintensität und/oder Helligkeit führen, durch eine vom Benutzer wählbare Gewichtung oder Maß berechnet werden.

8. Detektionsvorrichtung (10) nach einem der Ansprüche 1 bis 7, **dadurch gekennzeichnet, dass** durch die Frequenzanalysevorrichtung (16) eine Aufteilung der durch die Elektroden (12a, 12b) erfassten Aktionspotentiale in ihre Frequenzanteile erfolgt, wobei die Aufteilung insbesondere durch eine Fourier-Analyse, insbesondere mittels einer Fast Fourier Transformation oder Wavelets erfolgt.

9. Detektionsvorrichtung (10) nach einem der Ansprüche 1 bis 6, **dadurch gekennzeichnet, dass** die Detektionsvorrichtung eine Vielzahl von Elektroden (12a, 12b) aufweist.

10. Detektionsvorrichtung (10) nach einem der Ansprüche 1 - 9, **dadurch gekennzeichnet, dass** insbesondere unter Verwendung des Elektrodenabstands eine Ermittlung der Tiefe einer Nervenfaser erfolgt.

11. Detektionsvorrichtung (10) nach einem der Ansprüche 1 - 10, **dadurch gekennzeichnet, dass** die Energie, die zwischen 0 und 1.000 Hz liegt, in einer ersten Farbe auf der Anzeigevorrichtung angezeigt wird, und die Energie, die zwischen 1.000 und 10.000 Hz liegt, in einer zweiten Farbe auf der Anzeigevorrichtung angezeigt wird.
